# EUROPEAN PATENT SPECIFICATION

(11) **EP 0 619 324 B1**
(45) Date of publication and mention of the grant of the patent: **03.07.2002**
(21) Application number: 92924918.3
(22) Date of filing: 15.12.1992
(51) Int. Cl.: C12N 15/13, C07K 14/28, C12P 21/02, C12P 21/08, C12N 5/10, C12N 15/62, A61K 39/395

(54) **HUMAN TYPE ANTIBODY REACTIVE WITH GPIIb/IIIa**
MIT GP11B/IIIA REAKTIVE HUMANE ANTIKÖRPER
ANTICORPS DE TYPE HUMAIN REACTIF A GPIIb/IIIa

(30) Priority: 20.12.1991 US 812111; 09.06.1992 US 895952; 11.09.1992 US 944159
(43) Date of publication of application: 12.10.1994
(73) Proprietor: YAMANOUCHI PHARMACEUTICAL CO. LTD., Tokyo 103 (JP); PROTEIN DESIGN LABS, INC., Fremont, CA 94555 (US)
(72) Inventor: CO, Man, Sung, Cupertino, CA 95014 (US); TSO, J., Yun, Menlo Park, CA 94025 (US)
(74) Representative: Geering, Keith Edwin
(86) International application number: JP9201630
(87) International publication number: WO9313133

(56) References cited:
- EP-A- 0 255 694
- JP-A- 62 296 890
- ABSTRACTS SELECTED FOR PRESENTATION AT THE XIIITH CONGRESS OF THE EUROPEAN SOCIETY OF CARDIOLOGY, AMSTERDAM, NETHERLANDS, AUGUST 18-22, 1991. EUR HEART J, 12 (ABSTR. SUPPL.). 1991. 26., BHATTACHARYA S ET AL 'CHIMERIC HUMANIZED MONOCLONAL ANTIPLATELET ANTIBODY 7E3 PRODUCES PROLONGED DOSE-DEPENDENT INHIBITION OF PLATELET FUNCTION'
- J BIOL CHEM, JAN 5 1989, 264 (1) P259-65, UNITED STATES, TAUB R ET AL 'A monoclonal antibody against the platelet fibrinogen receptor contains a sequence that mimics a receptor recognition domain in fibrinogen.'
- J AUTOIMMUN, JUN 1991, 4 (3) P433-46, ENGLAND, KUNICKI TJ ET AL 'Nucleotide sequence of the human autoantibody 2E7 specific for the platelet integrin IIb heavy chain.'
- Thrombosis Research, Vol. 38, No. 5, (1985), D. HEINRICH et al., "Monoclonal antibodies against human platelet membrane glycoproteins IIb/IIIa 2. Different effects on Platelet function", p. 547-559.
- Blood, Vol. 68, No. 3, (1986), B.S. COLLER et al., "Artithrombotic effect of a monoclonal antibody to the platelet glycoprotein IIb/IIIa receptor in an experimental animal model", p. 783-786.
- Blood, Vol. 70, No. 1, (1987), D.J. NUGENT et al., "A human monoclonal autoantibody recognizes a neoantigen on glycoprotein IIIa expressed on stored and activated platelets", p. 16-22.
- Journal of Biological Chemistry, Vol. 258, No. 20, (1983), D. PIDARD et al., "Interaction of AP-2, a monoclonal antibody specific for the human platelet glycoprotein IIb-IIIa complex, with intact platelets", p. 12582-12586.
- Journal of Clinical Investigation, Vol. 72, No. 1, (1983), B.S. COLLER et al., "A murine monoclonal antibody that completely blocks the binding of fibrinogen of platelets produces a thrombasthenic-like state in normal platelets and binds to glycoproteins IIb and/or IIIa", p. 325-338.
- Proceedings of the National Academy of Sciences of the U.S.A., Vol. 86, No. 24, (1989), C. QUEEN et al., "A humanized antibody that binds to the interleukin 2 receptor", p. 10029-10033.
- Proceedings of the National Academy of Sciences of the U.S.A., Vol. 88, No. 7, (1991), M.S. CO et al., "Humanized antibodies for antiviral therapy", p. 2869-2873.

## Description

### Technical Field

The present invention relates generally to the combination of recombinant DNA and monoclonal antibody technologies for developing novel biologics and, more particularly, for example, to the production of non-immunogenic (in humans) immunoglobulins specific for the GPIIb/IIIa antigen and their uses in vitro and in vivo.

### Background Art

Cardiovascular disease is a leading cause of death and disability in developed countries. Atherosclerosis, or narrowing and hardening of the arteries, is a major contributor to cardiovascular disease. Arterial thrombosis, the formation of a blood clot or thrombus, commonly occurs in arteries effected by atherosclerosis, especially when a rupture occurs in a plaque that has built up on the vessel wall. The formation of a thrombus in a coronary artery can cause an acute myocardial infarction (heart attack) if the blockage is sustained, or unstable angina if the blockage is transient. A thrombus in a cerebral artery can cause a stroke or a transient ischemic attack. An arterial thrombus is composed of an aggregate of platelets together with the protein fibrin.

The glycoprotein GPIIb/IIIa is found on the surface of platelets and plays a key role in their aggregation and hence the formation of thrombi (see, generally, Phillips et al., Blood, 71, 831 (1988), which is incorporated herein by reference). The GPIIb/IIIa complex is composed of one molecule of GPIIb (M.W. = 140 kDa), which itself consists of a large (M.W. = 125 kDa) chain and a small (M.W. = 25 kDa) chain linked by one or more disulfide bonds and one molecule of GPIIIa (M.W. = 105 kDa), which is a single polypeptide chain. Ca²⁺ ions are required to maintain the heterodimeric structure of GPIIb/IIIa, which is a member of the integrin family of adhesion molecules. When platelets are activated by a physiological agonist such as thrombin, adenosine diphosphate (ADP) or epinephrine, the structure or environment of the GPIIb/IIIa molecules on the surface of the platelets is altered so they can bind the serum protein fibrinogen. The fibrinogen cross-links the platelets so they form aggregates. GPIIb/IIIa is also a receptor for the adhesive proteins fibronectin, von Willebrand factor and vitronectin, and therefore contributes to the adhesion and spreading of platelets on the subendothelium of injured blood vessels.

A number of monoclonal antibodies have been developed that bind to GPIIb/IIIa and block its ability to bind fibrinogen. These antibodies include mouse monoclonal antibody 10E5 (Coller et al., J. Clin. Invest., 72, 325 (1983)), mouse monoclonal antibody AP-2 (Pidard et al., J. Biol. Chem., 258, 12582 (1983)) and mouse monoclonal antibody 7E3 (Coller, J. Clin. Invest., 76, 101 (1985), which are incorporated herein by reference) and C4G1, described herein. By blocking fibrinogen binding, these antibodies inhibit the aggregation of platelets in vitro in response to agonists such as ADP. Anti-GPIIb/IIIa antibodies may therefore inhibit the formation of thrombi by preventing the aggregation of additional platelets. In fact, treatment with the 7E3 antibody has been found to contribute to recanalization of the coronary artery in a canine model of coronary arterial thrombosis, especially when administered in combination with tissue plasminogen activator (tPA) (Gold et al., Circulation, 77, 670 (1988)).

Unfortunately, the use of non-human monoclonal antibodies such as 7E3 or C4G1 have certain drawbacks in human treatment, particularly in repeated therapeutic regimens as explained below. Mouse monoclonal antibodies, for example, tend to have a short half-life in humans, and lack other important immunoglobulin functional characteristics when used in humans.

Perhaps more importantly, non-human monoclonal antibodies contain substantial stretches of amino acid sequences that will be immunogenic when injected into a human patient. Numerous studies have shown that, after injection of a foreign antibody, the immune response elicited by a patient against an antibody can be quite strong, essentially eliminating the antibody's therapeutic utility after an initial treatment. Moreover, as increasing numbers of different mouse or other antigenic (to humans) monoclonal antibodies can be expected to be developed to treat various diseases, after the first or several treatments with any different non-human antibodies, subsequent treatments even for unrelated therapies can be ineffective or even dangerous in themselves, because of cross-reactivity.

While the production of so-called "chimeric antibodies" (e.g., mouse variable regions joined to human constant regions) has proven somewhat successful in avoiding the aforementioned reactivity against foreign antibodies, a significant immunogenicity problem remains. In general, the production of human immunoglobulins reactive with GPIIb/IIIa antigen with high affinity, as with many antigens, would be extremely difficult using typical human monoclonal antibody production techniques. To date, no information is available concerning human immunoglobulins which are capable of inhibiting the aggregation of platelets and are useful in the treatment of thrombosis as therapeutic agents. Similarly, utilizing recombinant DNA technology to produce so-called "humanized" or "reshaped" antibodies (see, e.g., Riechmann et al., Nature, 332, 323 (1988) and EPO Publication No. 0239400, which are incorporated herein by reference), provides uncertain results, in part due to unpredictable binding affinities.

J.Biol.Chem. vol 264, No.1, pp 259-265, 1989, discloses a murine monoclonal antibody PAC1 which binds to GPIIb/IIIa only on activated platelets and inhibits platelet aggregation induced by agonists.

There is a need for improved forms of humanized immunoglobulins specific for GPIIb/IIIa antigen that are substantially non-immunogenic in humans, yet easily and economically produced in a manner suitable for therapeutic formulation and other uses. The present invention fulfills these and other needs.

### Disclosure of the Invention

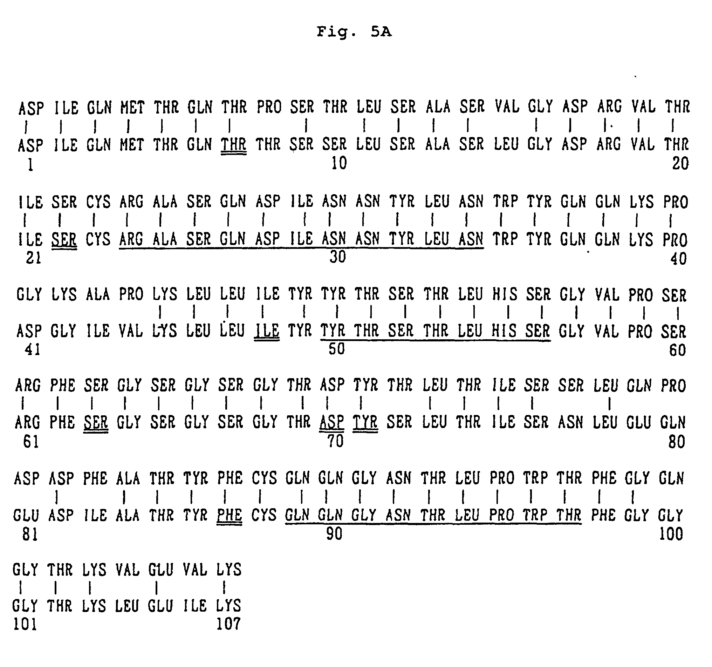

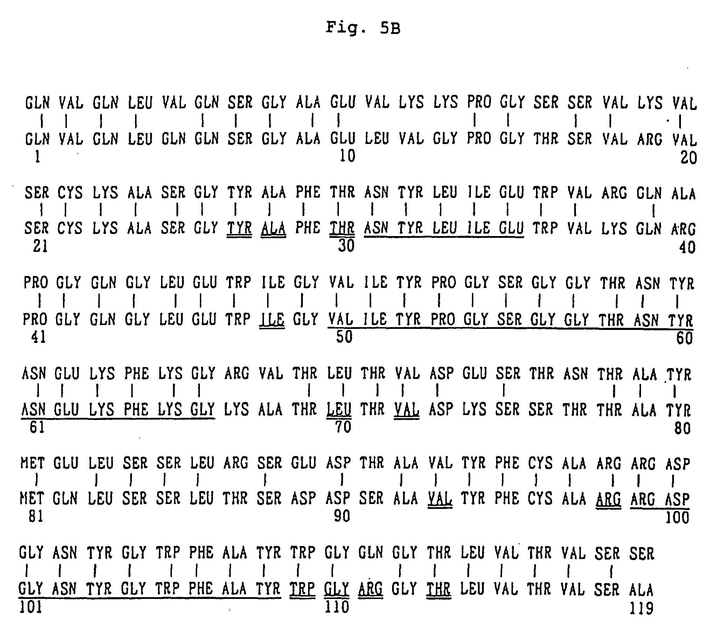

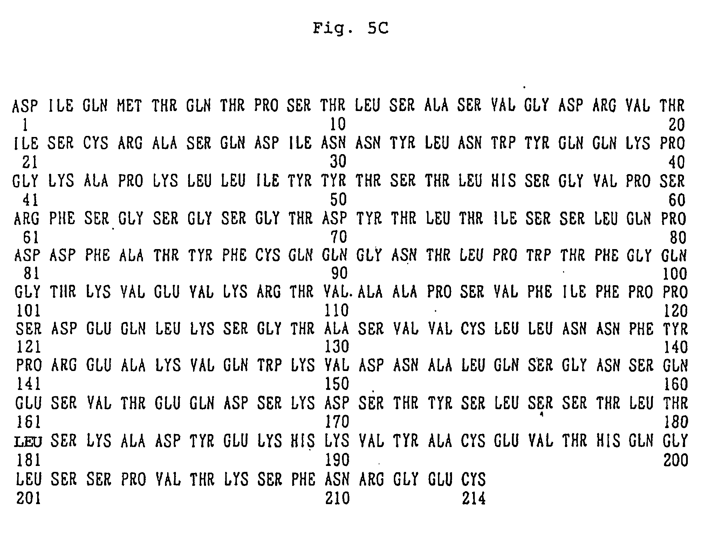

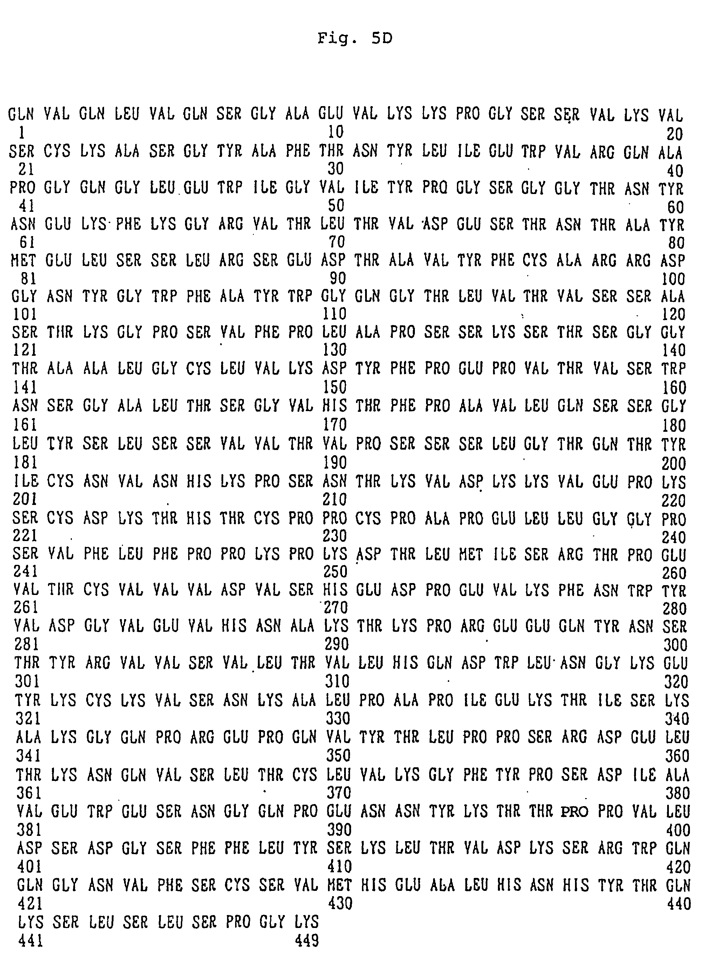

The present invention provides a humanized immunoglobulin having the properties [1] an epitope for said humanized immunoglobulin exists in a glycoprotein GPIIb/IIIa on human platelets,[2] an epitope for said humanized immunoglobulin does not exist in human vascular endothelial cells, [3] said humanized immunoglobulin has a binding affinity of 10⁷ M⁻¹ or stronger, and [4] said humanized immunoglobulin is capable of blocking the aggregation of human platelets in response to agonists, wherein said humanized immunoglobulin comprises a sequence which is the mature light variable region of the upper sequence in Figure 5A and a sequence which is the mature heavy variable region of the upper sequence in Figure 5B; preferably said humanized immunoglobulin comprises a sequence which is the mature light region as shown in Figure 5C and a sequence which is the mature heavy region as shown in Figure 5D. It also provides polynucleotides coding for said mature light variable or said mature heavy variable regions of such humanized immunoglobulin, polynucleotides coding for said mature light region of Fig.5C or for said mature heavy region of Fig.5D, expression vectors comprising such polynucleotides, cells transfected with such expression vectors, and processes for producing such humanized immunoglobulin by culturing said cells. It further provides compositions, containing humanized immunoglobulins specifically capable of binding to GPIIb/IIIa antigen, which are useful in the treatment of arterial thrombosis-related human disorders. The immunoglobulins can have two pairs of light chain/heavy chain complexes, at least one chain comprising one or more mouse complementarity determining regions (CDR) functionally joined to human framework region segments. For example, mouse complementarity determining regions, with or without additional naturally-associated mouse amino acid residues, can be introduced into human framework regions to produce humanized immunoglobulins capable of binding to the GPIIb/IIIa antigen at affinity levels stronger than about 10⁷ M⁻¹. These humanized immunoglobulins will also be capable of blocking the binding of the CDR-donating mouse monoclonal antibody to GPIIb/IIIa.

The immunoglobulins, including binding fragments and other derivatives thereof, of the present invention may be produced readily by a variety of recombinant DNA techniques, with ultimate expression in transfected cells, preferably immortalized eukaryotic cells, such as myeloma or hybridoma cells. Polynucleotides comprising a first sequence coding for humanized immunoglobulin framework regions and a second sequence set coding for the desired immunoglobulin complementarity determining regions can be produced synthetically or by combining appropriate cDNA and genomic DNA segments.

The humanized immunoglobulins may be utilized alone in substantially pure form, or together with a therapeutic agent such as tissue plasminogen activator or aspirin active against thrombi. All of these compounds will be particularly useful in treating acute myocardial infarction, unstable angina, stroke and other platelet-mediated disorders. The humanized immunoglobulins or their complexes can be prepared in a pharmaceutically accepted dosage form, which will vary depending on the mode of administration.

The humanized immunoglobulins may also be utilized together with a labeling moiety for use in diagnosing the presence and location of a thrombus, or certain types of cancer cells which develop GPIIb/IIIa on their surfaces, in a human patient. Such labeling moieties include, but are not limited to, radiopaque dyes, radiocontrast agents, fluorescent molecules, spin-labeled molecules, enzymes, or other labeling moieties of diagnostic value, particularly in radiologic or magnetic resonance imaging techniques.

### Brief Description of the Figures

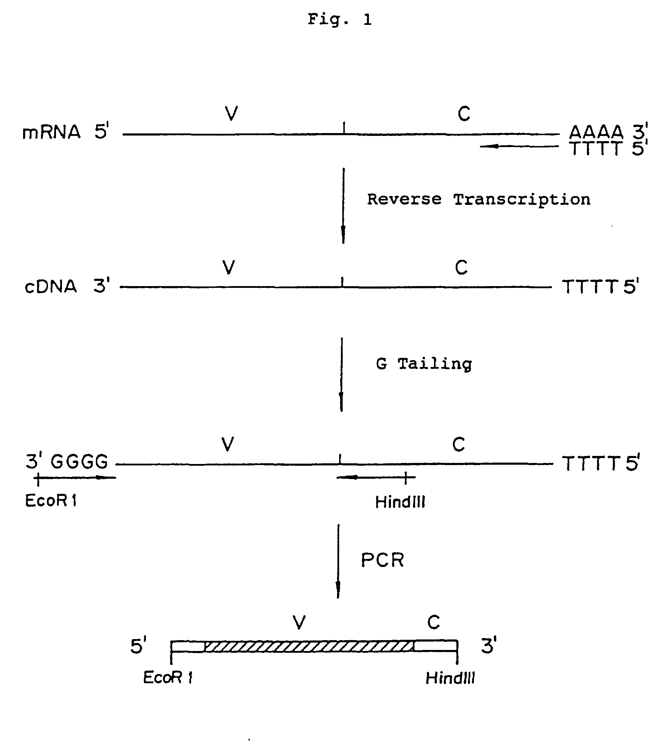

Figure 1. Scheme for anchored polymerase chain reaction (PCR) cloning of the heavy and light chain variable domain cDNAs. RNA was prepared from about 10⁷ hybridoma cells using the hot phenol extraction method. Briefly, cells were resuspended and vortexed in 1 ml of RNA extraction buffer (50 mM sodium acetate pH 5.2/1% SDS), extracted with 0.5 ml of phenol pH 5.2 at 65°C for 15 min, followed by another 15 min on ice. The aqueous phase was recovered and precipitated twice with ethanol. cDNA was synthesized from 10 µg of total RNA using reverse transcriptase (BRL, Bethesda, MD) and oligo dT₁₂₋₁₈ (Pharmacia, Piscatway, NJ) as primers. A poly(dG) tail was attached to the 3' end of the cDNA using terminal deoxynucleotide transferase (BRL) (E.Y. Loh et al., Science, 243, 217 (1989)). The variable domain genes (V) were amplified using AmpliTaq (Perkin Elmer-Cetus) with the primer mc045 (TAATCTAGAATTCCCCCCCCCCCCCCCCC) that hybridized to the poly(dG) tails and primers that hybridized to the constant region genes (C). For the light chain, the primer used was mc046 (TATAGAGCTCAAGCTTGGATGGTGGGAAGATGGATACAGTTGGTGC). For the heavy chain, the primer used was mc047 (TATAGAGCTCAAGCTTCCAGTGGATAGAC(CAT)GATGGGG(GC)TGT(TC)GTTTTGGC). The sequence in parenthesis indicates a base degeneracy. The degeneracy was introduced so that the primer would be able to hybridize to most gamma chains. The amplified fragments were then digested with EcoRI and HindIII and cloned into pUC18 vector for sequencing.

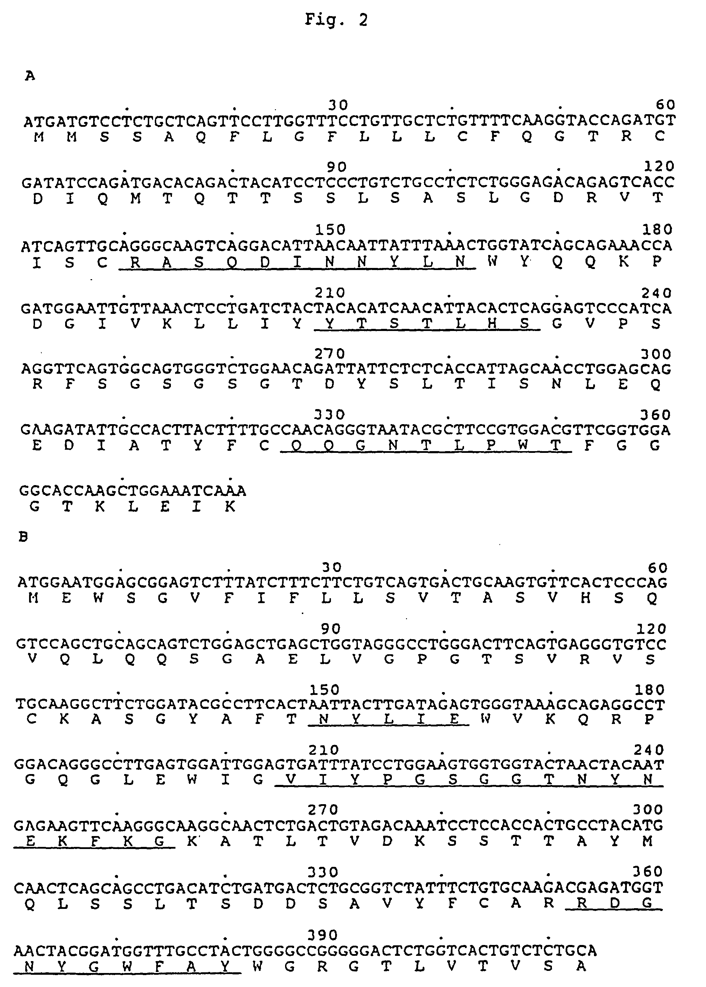

Figure 2. Sequences of the cDNA and translated amino acid sequences of the light chain (Fig. 2A) and heavy chain (Fig. 2B) variable regions of the antibody C4G1. The CDR sequences are underlined. The mature light chain protein begins with amino acid 21 Asp and the mature heavy chain protein with amino acid 20 Gln, preceded by the respective signal sequences.

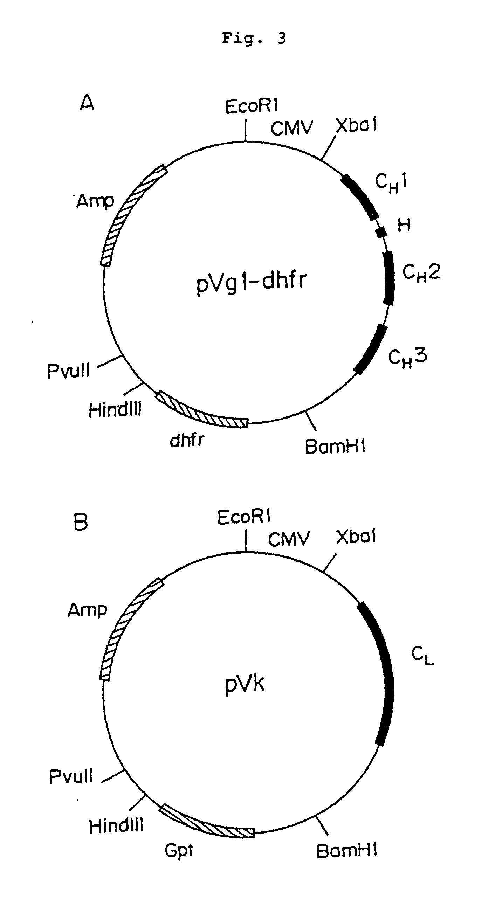

Figure 3. Schematic diagram of the plasmids pVg1-dhfr (Fig. 3A) and pVk (Fig. 3B). The plasmid pVg1-dhfr contains the following parts: an approximately 4200 base pair (bp) BamHI-EcoRI fragment containing the amp and dhfr genes; a 630-bp fragment containing the human cytomegalovirus (CMV) IE1 gene promoter and enhancer (Boshart et al., Cell, 41, 521 (1985), which is incorporated herein by reference) flanked at the 5' and 3' ends by EcoRI and XbaI linkers respectively; and a 2800-bp HindIII-PvuII fragment containing the human gamma-1 constant region gene with 215-bp of the preceding intron and the poly(A) signal (Ellison et al., Nucleic Acids Res., 10, 4071 (1982), which is incorporated herein by reference). The plasmid pVk was similarly constructed, with a 1530-bp EcoRI-XbaI fragment containing the human kappa constant region gene and about 335 bp of the preceding intron (Hieter et al., Cell, 22, 197 (1980), which is incorporated herein by reference) replacing the gamma-1 gene; and the gpt gene replacing the dhfr gene. The fragments containing the human gamma-1 and kappa constant region genes were flanked at their 5' and 3' ends respectively by created XbaI and BamHI sites. The plasmids were constructed from the indicated parts using methods well-known in the art (see, Maniatis et al., Molecular Cloning: A Laboratory Manual, 2nd Ed., Cold Spring Harbor Laboratory, Cold Spring Harbor, N.Y.) For example, pVg1-dhfr was constructed from the plasmid pVγ1 (Queen et al., Proc. Natl. Acad. Sci. USA, 86, 10029 (1989)) by replacing the HindIII-BglII fragment containing the hyg gene with a 660 bp fragment containing the dhfr gene and extending to a BglII site (Simonsen et al., Proc. Natl. Acad. Sci. USA, 80, 2495 (1983)), and the EcoRI-XbaI fragment containing the immunoglobulin promoter and enhancer with the fragment containing the CMV promoter and enhancer.

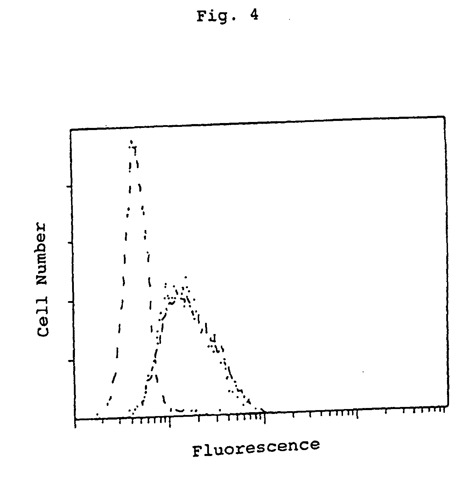

Figure 4. Fluorocytometry of HEL 92.1.7 cells stained with (---) no antibody, (...) chimeric C4G1 antibody, (―) humanized C4G1 antibody. Cells were suspended in FACS buffer (PBS + 2% FCS + 0.1% sodium azide) at approximately 5×10⁶/ml. 100 µl of cell suspension was transferred to a polystyrene tube and incubated with 80 ng of purified antibody on ice for 30 min. The cells were washed with FACS buffer and incubated with FITC labeled goat anti-human Ig antibody on ice for another 30 min. The cells were washed again and finally resuspended in PBS + 1% paraformaldehyde. Cells were analyzed on a FACSmate (Becton Dickinson).

Figure 5. Amino acid sequences of the light chain (Fig. 5A) and the heavy chain (Fig. 5B) variable regions of the humanized C4G1 antibody (upper lines) and mouse C4G1 antibody (lower lines), not including signal sequences. The three CDR's in each chain are underlined. Residues in the framework that have been replaced with mouse amino acids or consensus human amino acids in the humanized antibody are double underlined. Amino acid sequences of the complete light chain (Fig. 5C) and heavy chain (Fig. 5D) of the humanized C4G1 antibody.

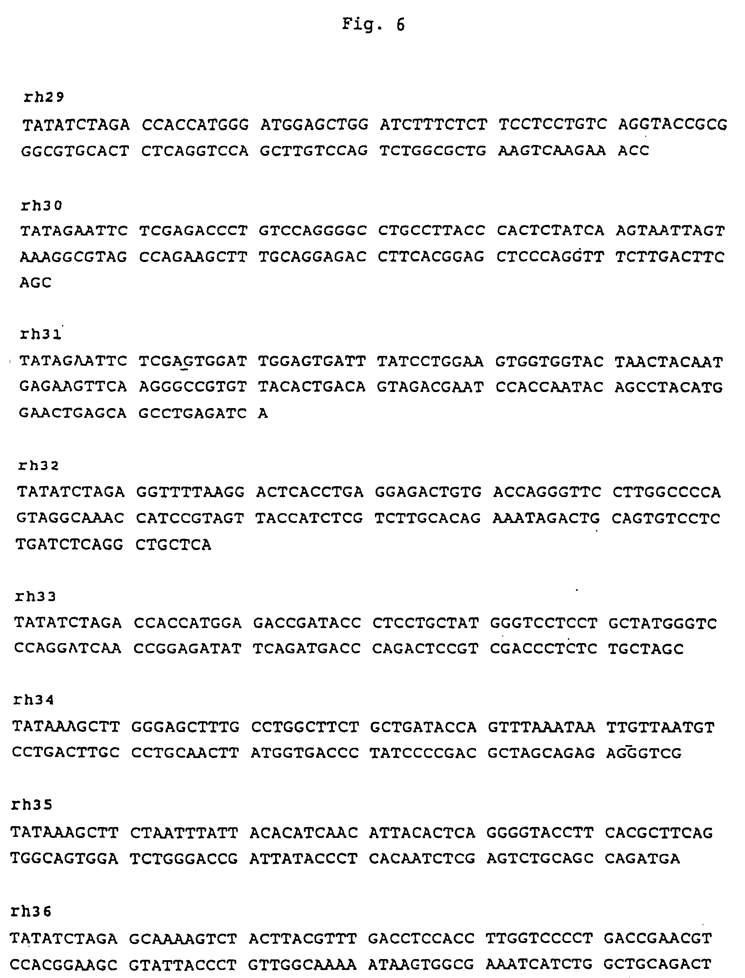

Figure 6. Oligonucleotides used in the construction of the humanized C4G1 heavy chain (rh29-rh32) and light chain (rh33-rh36). The following pairs of oligonucleotides were mixed, extended with Klenow polymerase or AmpliTaq and cut with the indicated enzymes before ligation into pUC18: rh29 and rh30 with XbaI and EcoRI, rh31 and rh32 with XbaI and EcoRI, rh33 and rh34 with XbaI and HindIII, rh35 and rh36 with XbaI and HindIII. Then the rh29-rh-30 and rh31-rh32 fragments were excised from pUC18 with XbaI and XhoI and ligated together into the XbaI site of pVg1-dhfr; and the rh33-rh34 and rh35-rh36 fragments were excised with XbaI and HindIII and ligated together into the XbaI site of pVk.

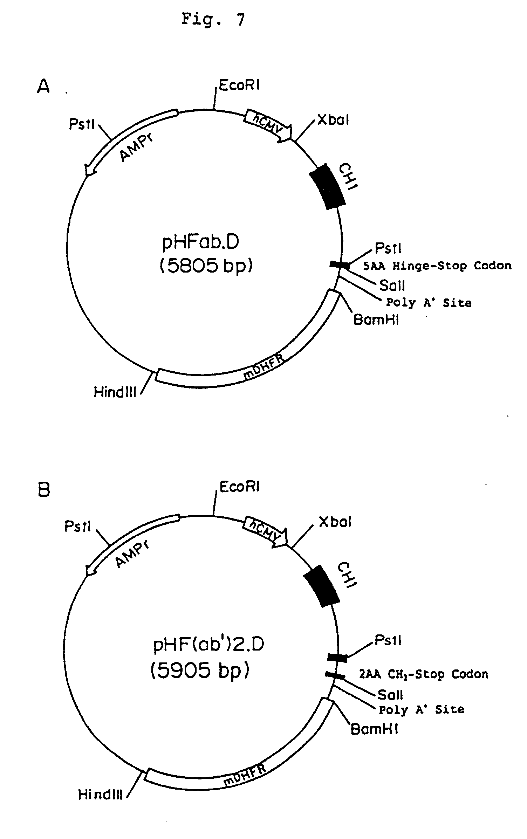

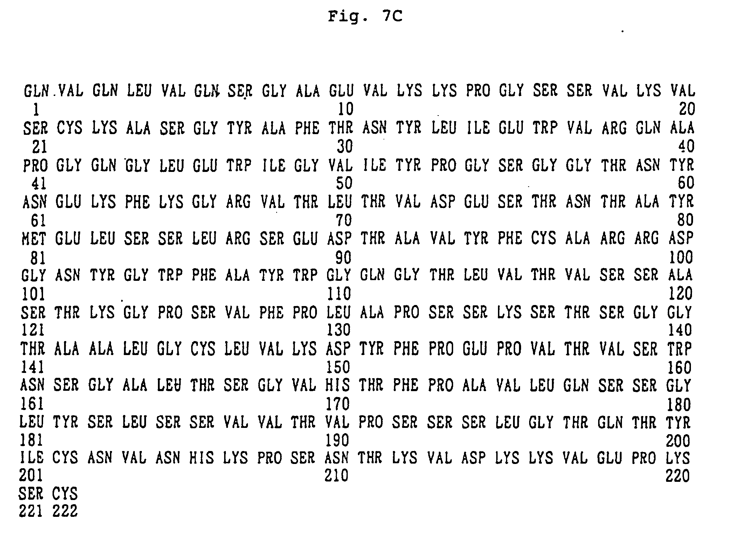

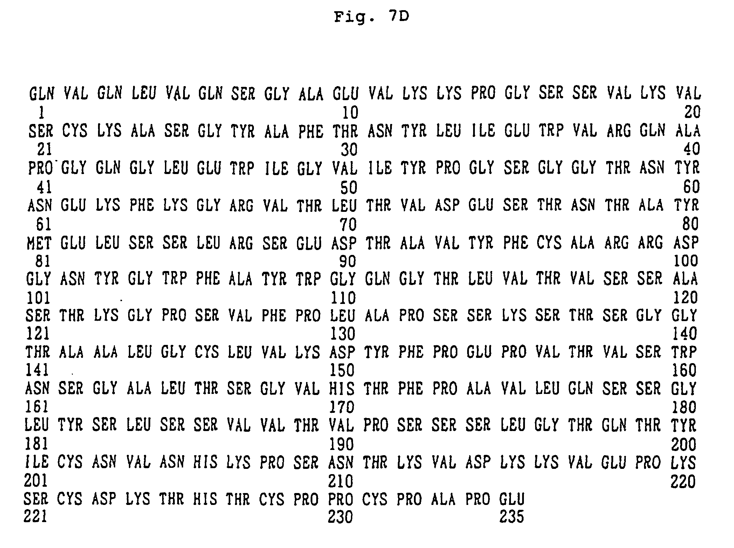

Figure 7. Schematic diagrams of the plasmids pHFab.D (Fig. 7A) and pHF(ab')2.D (Fig. 7B), used respectively for expression of heavy chains of the humanized Fab and F(ab')₂ fragments. The plasmids are similar to the plasmid pVg1-dhfr (Fig. 3) but pHFab.D contains only C_{H}1 and the first 5 amino acids of the hinge exon of the human Cγ1 gene, followed by a stop codon and splice donor signal; and pHF(ab')2.D contains only C_{H}1, the hinge and the first 2 amino acids of C_{H}2, followed by a stop codon and splice donor signal. The final exon is followed by a region from beyond the mouse γ2a constant region gene that contains a poly A signal. Amino acid sequences of the heavy chain of the Fab fragment (Fig. 7C) and recombinant F(ab')₂ fragment (Fig. 7D), designated F(ab')₂-1, of the humanized C4G1 antibody.

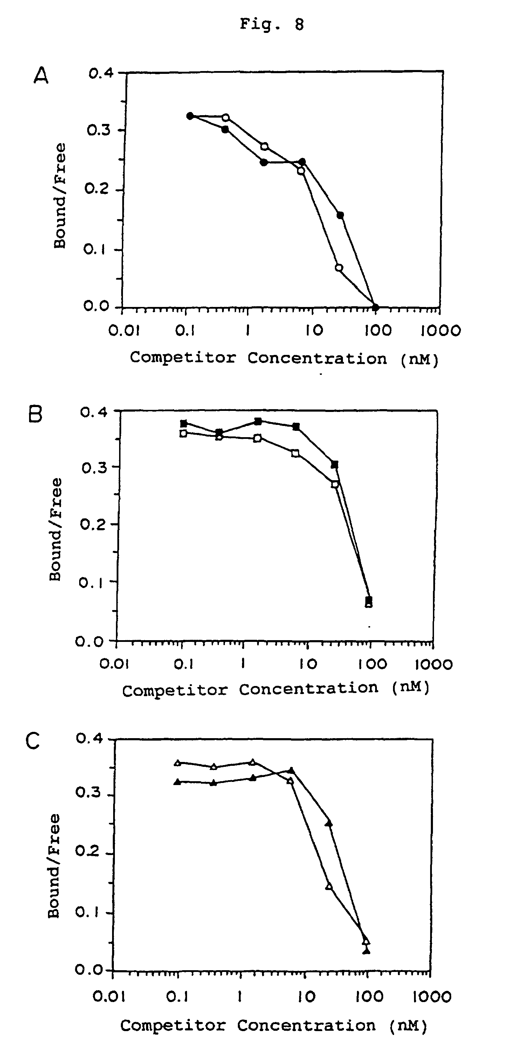

Figure 8. Competitive binding of humanized and mouse C4G1 antibodies and fragments to platelets. About 3×10⁷ platelets were incubated with 4.5 ng of radio-iodinated mouse C4G1 tracer antibody (3.5 µCi/µg) and varying amounts of either unlabeled mouse C4G1 antibody or fragments (closed symbols) or humanized C4G1 antibody or fragments (open symbols). (A) Intact antibodies, (B) Fab fragments, (C) Mouse F(ab')₂ and humanized F(ab')₂-1 fragments.

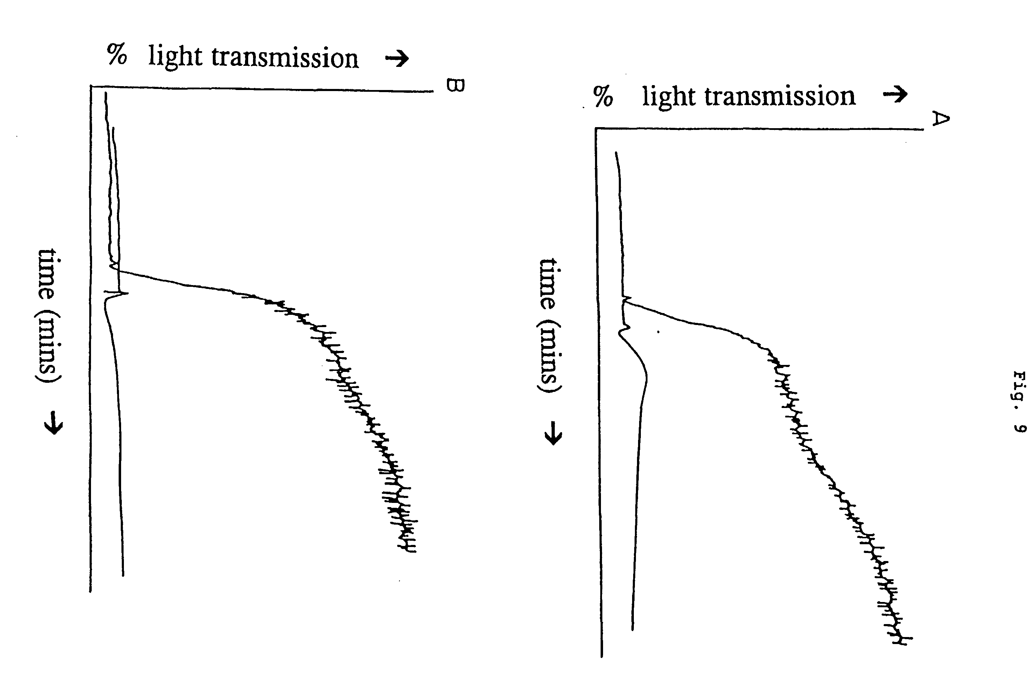

Figure 9. Inhibition of platelet aggregation by mouse (A) and humanized (B) C4G1 antibodies. The Y-axis shows percent light transmission; the X-axis shows time in minutes. The dark upper curve in each panel shows the increase in light transmission caused by platelet aggregation when no antibody is added; the lighter lower curves show that addition of antibodies strongly inhibits platelet aggregation, as measured by change in light transmission.

### Best Mode for Carrying out the Invention

In accordance with the present invention, humanized immunoglobulins specifically reactive with GPIIb/IIIa related epitopes are provided. These immunoglobulins, which have, binding affinities to GPIIb/IIIa of at least about 10⁷ M⁻¹, and preferably 10⁸ M⁻¹ to 10¹⁰ M⁻¹ or stronger, are capable of preventing platelet aggregation. The humanized immunoglobulins will have a human framework and will have one or more complementarity determining regions (CDR's) from an immunoglobulin, typically a mouse immunoglobulin, specifically reactive with GPIIb/IIIa antigen. In a preferred embodiment, one or more of the CDR's will come from the C4G1 antibody. Thus, the immunoglobulins of the present invention, which can be produced economically in large quantities, find use, for example, in the treatment of platelet-mediated disorders in human patients by a variety of techniques.

The basic antibody structural unit is known to comprise a tetramer. Each tetramer is composed of two identical pairs of polypeptide chains, each pair having one "light" (about 25 kD) and one "heavy" chain (about 50-70 kD). The amino-terminal portion of each chain includes a variable region of about 100 to 110 or more amino acids primarily responsible for antigen recognition. The carboxy-terminal portion of each chain defines a constant region primarily responsible for effector function.

Light chains are classified as either kappa (κ) or lambda (λ). Heavy chains are classified as gamma (γ), mu (µ), alpha (α), delta (δ), or epsilon (∈), and define the antibody's isotype as IgG, IgM, IgA, IgD and IgE, respectively. Within light and heavy chains, the variable and constant regions are joined by a "J" region of about 12 or more amino acids, with the heavy chain also including a "D" region of about 10 or more amino acids. (see, generally, Fundamental Immunology, Paul, W., Ed., Chapter 7, pgs. 131-166, Raven Press, N.Y. (1984), which is incorporated herein by reference).

The variable regions of each light/heavy chain pair form the antibody binding site. The chains all exhibit the same general structure of relatively conserved framework regions joined by three hypervariable regions, also called Complementarity Determining Regions or CDR's (see, "Sequences of Proteins of Immunological Interest", Kabat, E., et al., U.S. Department of Health and Human Services, (1987); and Chothia and Lesk, J. Mol. Biol., 196, 901-917 (1987), which are incorporated herein by reference). The CDR's from the two chains of each pair are aligned by the framework regions, enabling binding to a specific epitope.

As used herein, the term "immunoglobulin" refers to a protein consisting of one or more polypeptides substantially encoded by immunoglobulin genes. The recognized immunoglobulin genes include the kappa, lambda, alpha, gamma, delta, epsilon and mu constant region genes, as well as the myriad immunoglobulin variable region genes. The immunoglobulins may exist in a variety of forms besides antibodies; including, for example, Fv, Fab, and F(ab')₂ as well as bifunctional hybrid antibodies (e.g., Lanzavecchia et al., Eur. J. Immunol., 17, 105 (1987)) and in single chains (e.g., Huston et al., Proc. Natl. Acad. Sci. USA, 85, 5879-5883 (1988) and Bird et al., Science, 242, 423-426 (1988), which are incorporated herein by reference). (see, generally, Hood et al., Immunology, Benjamin, N.Y., 2nd Ed. (1984), Harlow and Lane, Antibodies, A Laboratory Manual, Cold Spring Harbor Laboratory (1988) and Hunkapiller and Hood, Nature, 323, 15-16 (1986), which are incorporated herein by reference).

Chimeric antibodies are antibodies whose light and heavy chain genes have been constructed, typically by genetic engineering, from immunoglobulin gene segments belonging to different species. For example, the variable (V) segments of the genes from a mouse monoclonal antibody may be joined to human constant (C) segments, such as γ1 and γ3. A typical therapeutic chimeric antibody is thus a hybrid protein consisting of the V or antigen-binding domain from a mouse antibody and the C or effector domain from a human antibody, although other mammalian species may be used.

As used herein, the term "framework region" refers to those portions of immunoglobulin light and heavy chain variable regions that are relatively conserved (i.e., other than the CDR's) among different immunoglobulins in a single species, as defined by Kabat, et al., op. cit. As used herein, a "human framework region" is a framework region that is substantially identical (about 85% or more) to the framework region of a naturally occurring human antibody.

As used herein, the term "humanized immunoglobulin" refers to an immunoglobulin comprising a human framework, at least one CDR from a non-human antibody, and in which any constant region present is substantially identical to a human immunoglobulin constant region, i.e., at least about 85-90%, preferably at least 95% identical. Hence, all parts of a humanized immunoglobulin, except possibly the CDR's, are substantially identical to corresponding parts of one or more native human immunoglobulin sequences. For example, a humanized immunoglobulin would not encompass a chimeric mouse variable region/human constant region antibody.

Humanized antibodies have at least three potential advantages over mouse and in some cases chimeric antibodies for use in human therapy:
1) Because the effector portion is human, it may interact better with the other parts of the human immune system (e.g., destroy the target cells more efficiently by complement-dependent cytotoxicity (CDC) or antibody-dependent cellular cytotoxicity (ADCC)).
2) The human immune system should not recognize the framework or C region of the humanized antibody as foreign, and therefore the antibody response against such an injected antibody should be less than against a totally foreign mouse antibody or a partially foreign chimeric antibody.
3) Injected mouse antibodies have been reported to have a half-life in the human circulation much shorter than the half-life of normal antibodies (Shaw, D. et al., J. Immunol., 138, 4534-4538 (1987)). Injected humanized antibodies will presumably have a half-life essentially identical to naturally occurring human antibodies, allowing smaller and less frequent doses to be given.

In one aspect, the present invention is directed to recombinant polynucleotides encoding the heavy and/or light chain CDR's from an immunoglobulin capable of binding to an epitope of GPIIb/IIIa antigen - monoclonal antibody C4G1. The polynucleotides encoding these regions will typically be joined to polynucleotides encoding appropriate human framework regions. As to the human framework region, a framework or variable region amino acid sequence of a CDR-providing non-human immunoglobulin is compared with corresponding sequences in a human immunoglobulin sequence collection, and a sequence having high homology is selected. Exemplary polynucleotides, which on expression code for the polypeptide chains comprising the heavy and light chain CDR's of monoclonal antibody C4G1 are included in Fig. 2. Due to codon degeneracy and non-critical amino-acid substitutions, other polynucleotide sequences can be readily substituted for those sequences, as detailed below. The design of humanized immunoglobulins may be carried out as follows.
(1) When an amino acid falls under the following category, the framework amino acid of a human immunoglobulin to be used (acceptor immunoglobulin) is replaced by a framework amino acid from a CDR-providing non-human immunoglobulin (donor immunoglobulin):
   (a) the amino acid in the human framework region of the acceptor immunoglobulin is unusual for human immunoglobulin at that position, whereas the corresponding amino acid in the donor immunoglobulin is typical for human immunoglobulin at that position;
   (b) the position of the amino acid is immediately adjacent to one of the CDR's; or
   (c) the amino acid is within about 3x10ₘ⁻¹⁰ (3 Å) of a CDR in a tertiary structure immunoglobulin model. (see, Queen et al., op. cit., and Co et al., Proc. Natl. Acad. Sci. USA, 88, 2869 (1991), respectively, both of which are incorporated herein by reference.)
(2) When each of the amino acid in the human framework region of the acceptor immunoglobulin and a corresponding amino acid in the donor immunoglobulin is unusual for human immunoglobulin at that position, such an amino acid is replaced by an amino acid typical for human framework region at that position. For a detailed description of the production of humanized immunoglobulins (see, Queen et al. (op. cit.) and Co et al. (op. cit.)).

The polynucleotides will typically further include an expression control polynucleotide sequence operably linked to the humanized immunoglobulin coding sequences, including naturally-associated or heterologous promoter regions. Preferably, the expression control sequences will be eukaryotic promoter systems in vectors capable of transforming or transfecting eukaryotic host cells, but control sequences for prokaryotic hosts may also be used. Once the vector has been incorporated into the appropriate host, the host is maintained under conditions suitable for high level expression of the nucleotide sequences, and, as desired, the collection and purification of the light chains, heavy chains, light/heavy chain dimers or intact antibodies, binding fragments or other immunoglobulin forms may follow.

The nucleic acid sequences of the present invention capable of ultimately expressing the desired humanized antibodies can be formed from a variety of different polynucleotides (genomic or cDNA, RNA, synthetic oligonucleotides, etc.) and components (e.g., V, J, D, and C regions), as well as by a variety of different techniques. Joining appropriate genomic and synthetic sequences is presently the most common method of production, but cDNA sequences may also be utilized (see, European Patent Publication No. 0239400 and Reichmann, L. et al., Nature, 332, 323-327 (1988), both of which are incorporated herein by reference).

Human constant region DNA sequences can be isolated in accordance with well known procedures.from a variety of human cells, but preferably immortalized B-cells (see, Kabat op. cit. and WP 87/02671). The CDR's for producing the immunoglobulins of the present invention will be similarly derived from monoclonal antibodies capable of binding to GPIIb/IIIa and produced in any convenient mammalian source, including mice, rats, rabbits, or other vertebrate capable of producing antibodies by well known methods. Suitable source cells for the polynucleotide sequences and host cells for immunoglobulin expression and secretion can be obtained from a number of sources, such as the American Type Culture Collection (Catalogue of Cell Lines and Hybridomas, Fifth Edition (1985), Rockville, Maryland, U.S.A., which is incorporated herein by reference).

Polypeptide fragments comprising only a portion of the primary antibody structure may be produced, which fragments possess one or more immunoglobulin activities (e.g., complement fixation activity). These polypeptide fragments may be produced by proteolytic cleavage of intact antibodies by methods well known in the art, or by inserting stop codons at the desired locations in the vectors pVk and pVg1-dhfr (Fig. 3) using site-directed mutagenesis, such as after CH1 to produce Fab fragments or after the hinge region to produce F(ab')₂ fragments. Single chain antibodies may be produced by joining VL and VH with a DNA linker (see Huston et al., op. cit., and Bird et al., op. cit.). Also because like many genes, the immunoglobulin-related genes contain separate functional regions, each having one or more distinct biological activities, the genes may be fused to functional regions from other genes to produce fusion proteins having novel properties.

As stated previously, the polynucleotides will be expressed in hosts after the sequences have been operably linked to (i.e., positioned to ensure the functioning of) an expression control sequence. These expression vectors are typically replicable in the host organisms either as episomes or as an integral part of the host chromosomal DNA. Commonly, expression vectors will contain selection markers, e.g., tetracycline or neomycin, to permit detection of those cells transformed with the desired DNA sequences (see, e.g., U.S. Patent 4,704,362, which is incorporated herein by reference).

E. coli is one prokaryotic host useful particularly for cloning the polynucleotides of the present invention. Other microbial hosts suitable for use include bacilli, such as Bacillus subtilus, and other enterobacteriaceae, such as Salmonella, Serratia, and various Pseudomonas species. In these prokaryotic hosts, one can also make expression vectors, which will typically contain expression control sequences compatible with the host cell (e.g., an origin of replication). In addition, any number of a variety of well-known promoters will be present, such as the lactose promoter system, a tryptophan (trp) promoter system, a beta-lactamase promoter system, or a promoter system from phage lambda. The promoters will typically control expression, optionally with an operator sequence, and have ribosome binding site sequences and the like, for initiating and completing transcription and translation.

Other microbes, such as yeast, may also be used for expression. Saccharomyces is a preferred host, with suitable vectors having expression control sequences, such as promoters, including 3-phosphoglycerate kinase or other glycolytic enzymes, and an origin of replication, termination sequences and the like as desired.

In addition to microorganisms, mammalian tissue cell culture may also be used to express and produce the polypeptides of the present invention (see, Winnacker, From Genes to Clones, VCH Publishers, N.Y., N.Y. (1987), which is incorporated herein by reference). Eukaryotic cells are actually preferred, because a number of suitable host cell lines capable of secreting intact immunoglobulins have been developed in the art, and include the CHO cell lines, various COS cell lines, HeLa cells, preferably myeloma cell lines, etc., or transformed B-cells or hybridomas. Expression vectors for these cells can include expression control sequences, such as an origin of replication, a promoter, an enhancer (Queen et al., Immunol. Rev., 89, 49-68 (1986), which is incorporated herein by reference), and necessary processing information sites, such as ribosome binding sites, RNA splice sites, polyadenylation sites, and transcriptional terminator sequences. Preferred expression control sequences are promoters derived from immunoglobulin genes, SV40, Adenovirus, Bovine Papilloma Virus, cytomegalovirus and the like.

The vectors containing the polynucleotide sequences of interest (e.g., the heavy and light chain encoding sequences and expression control sequences) can be transferred into the host cell by well-known methods, which vary depending on the type of cellular host. For example, calcium chloride transfection is commonly utilized for prokaryotic cells, whereas calcium phosphate treatment or electroporation may be used for other cellular hosts. (see, generally, Maniatis et al., Molecular Cloning: A Laboratory Manual, Cold Spring Harbor Press (1982), which is incorporated herein by reference).

Once expressed, the whole antibodies, their dimers, individual light and heavy chains, or other immunoglobulin forms of the present invention can be purified according to standard procedures of the art, including ammonium sulfate precipitation, affinity columns, column chromatography, gel electrophoresis and the like (see, generally, Scopes, R., Protein Purification, Springer-Verlag, N.Y. (1982), which is incorporated herein by reference). Substantially pure immunoglobulins of at least about 90 to 95% homogeneity are preferred, and 98 to 99% or more homogeneity most preferred, for pharmaceutical uses. Once purified, partially or to homogeneity as desired, the polypeptides may then be used therapeutically (including extracorporeally) or in developing and performing assay procedures, immunofluorescent stainings, and the like. (see, generally, Immunological Methods, Vols. I and II, Lefkovits and Pernis, Eds., Academic Press, New York, N.Y. (1979 and 1981)).

The immunoglobulins of the present invention will typically find use individually in treating cardiovascular diseases and other thromboembolitic diseases. For example, typical disease states suitable for treatment include acute myocardial infarction, unstable angina, stroke, transient ischemic episodes, deep vein thrombosis and pulmonary embolism (see, generally, Hoffbrand & Pettit, Essential Haematology, Blackwell Scientific Publications, Oxford (1980)). The immunoglobulins may also be used to prevent reocclusion after angioplasty procedures or blood vessel operations and occlusion after or during extracorporeal blood circulation procedures such as dialysis, extracorporeal cardiopulmonary circulation and the like. The immunoglobulins of the present invention will also find use in the treatment of other platelet-mediated disorders, such as nephritis, peripheral circulatory failure, thromboangiitis obliterans, thrombosis after transplantation, chronic arterial obstruction, arteriosclerosis obliterans, coronary sclerosis, congestive heart failure, cerebrovascular spasm and the like. The immunoglobulins may also be used to treat thrombotic thrombocytopenic purpura, HUS and platelet-mediated autoimmune disease, to block metastasis, and to stimulate differentiation or growth of megakaryocytes or megakaryoblasts.

A CDR originated from the immunoglobulin which is capable of inhibiting the aggregation of platelets among GPIIb/IIIa-binding antibodies may be used preferably for the humanized immunoglobulin of the present invention. In addition, it is known that antibodies which react with vascular endothelial cells, such as 7E3 and the like antibodies, have a possibility of causing endothelial cell injury (Annals New York Academy of Science, 614, 204, (1991)). In consequence, it is more preferable to use a CDR originated from C4G1 or the like immunoglobulin so that the antigenic determinant bound by the antibody is present on platelets but is absent or present in reduced amounts on vascular endothelial cells. It is expected that a humanized immunoglobulin of the present invention, such as the humanized immunoglobulin that binds to the antigenic determinant identified by the mouse antibody C4G1, will not cause endothelial cell injury if administered.

In the case of the aggregation of platelets caused by the ADP or adrenaline stimulus, a reversible primary aggregation occurs after the addition of the agonist, followed by a secondary aggregation which is dose-dependently irreversible and is accompanied by a releasing reaction. It is known that 7E3 or the like antibody inhibits the aggregation of platelets when platelets are treated in advance with the antibody, but the antibody which inhibits the aggregation of platelets when added during the platelet aggregation process was not known. However, it was found that the C4G1 antibody inhibits not only the primary aggregation but also the secondary aggregation when the antibody was added after generation of the primary aggregation. In consequence, it is highly probable that a humanized immunoglobulin prepared making use of a CDR originated from the C4G1 antibody or an immunoglobulin having similar function to the C4G1 antibody will inhibit also initial steps of the activation and aggregation of platelets during the acute stage of thrombotic diseases.

It is known that levels of thromboxane A₂, PAI-1 and the like in blood increase rapidly after reconstruction of blood flow by means of PTCA, PTCR or the like for the treatment of thrombotic diseases. As a cause of this, activation of platelets and release of various mediators such as TGF-β₁, PDGF and the like are regarded as important factors. It was found when examined in vitro that the mouse C4G1 antibody is able to inhibit not only the aggregation of platelets caused by ADP stimulus but also releasing reaction of platelets. In consequence, it is probable that a humanized immunoglobulin prepared by making use of a CDR originated from an immunoglobulin which can inhibit both aggregation of platelets and releasing reaction of platelets, such as the mouse C4G1 antibody, will inhibit not only the aggregation of platelets but also the releasing reaction of platelets when used in humans, thus leading to a possibility of inhibiting side effects after a treatment by reconstruction of blood flow such as PTCA, PTCR or the like.

Any humanized immunoglobulins of the present invention may also be used in combination with other antibodies, particularly humanized antibodies reactive with different platelet antigens or clotting factors. For example, suitable antigens to which a cocktail of humanized immunoglobulins may react include VLA-2, VLA-5, GPIb, GPIV, von Willebrand factor, thrombin and the platelet thrombin receptor (see, Coller, New Eng. J. Med., 322, 33 (1990)).

The immunoglobulins can also be used as separately administered compositions given in conjunction with other therapeutic agents. Typically, the agents may include aspirin and heparin, but numerous additional agents (e.g., tPA) well-known to those skilled in the art for treatment of cardiovascular disease may also be utilized.

The humanized immunoglobulins and pharmaceutical compositions thereof of this invention are particularly useful for parenteral administration, i.e., subcutaneously, intramuscularly or intravenously. The compositions for parenteral administration will commonly comprise a solution of the immunoglobulin or a cocktail thereof dissolved in an acceptable carrier, preferably an aqueous carrier. A variety of aqueous carriers can be used, e.g., water, buffered water, 0.4% saline, 0.3% glycine, 5% glucose, human albumin solution and the like. These solutions are sterile and generally free of particulate matter. These compositions may be sterilized by conventional, well-known sterilization techniques. The compositions may contain pharmaceutically acceptable auxiliary substances as required to approximate physiological conditions such as pH adjusting and buffering agents, tonicity agents, toxicity adjusting agents and the like, for example, sodium acetate, sodium chloride, potassium chloride, calcium chloride, sodium lactate, sodium citrate, etc. The concentration of immunoglobulin in these formulations can vary widely, i.e., from less than about 0.5%, usually at least about 1% to as much as 15 or 20% by weight and will be selected primarily based on fluid volumes, viscosities, etc., in accordance with the particular mode of administration selected.

Thus, a typical pharmaceutical composition for injection could be made up to contain 1 ml sterile buffered water, and 1 to 10 mg of immunoglobulin. A typical composition for intravenous infusion could be made up to contain 250 ml of sterile Ringer's solution, and 150 mg of immunoglobulin. Actual methods for preparing parenterally administrable compositions will be known or apparent to those skilled in the art and are described in more detail in, for example, Remington's Pharmaceutical Science, 15th Ed., Mack Publishing Company, Easton, Pennsylvania (1980), which is incorporated herein by reference.

The immunoglobulins of this invention can be frozen or lyophilized for storage and reconstituted in a suitable carrier prior to use. This technique has been shown to be effective with conventional immunoglobulins and art-known lyophilization and reconstitution techniques can be employed. It will be appreciated by those skilled in the art that lyophilization and reconstitution can lead to varying degrees of immunoglobulin activity loss (e.g., with conventional immunoglobulins, IgM antibodies tend to have greater activity loss than IgG antibodies) and that use levels may have to be adjusted to compensate.

The compositions containing the present humanized immunoglobulins or a cocktail thereof can be administered for therapeutic or prophylactic treatments. In therapeutic application, compositions are administered to a patient already suffering from an arterial thrombosis or other platelet-mediated disease, in an amount sufficient to cure or at least partially arrest the disease and its complications. An amount adequate to accomplish this is defined as a "therapeutically effective dose". Amounts effective for this use will depend upon the severity of the disease and the general state of the patient's own immune system, but generally range from about 1 to about 200 mg of immunoglobulin per dose, with dosages of from 5 to 25 mg per patient being more commonly used. It must be kept in mind that the materials of this invention may generally be employed in serious disease states, that is, life-threatening or potentially life-threatening situations. In such cases, in view of the minimization of extraneous substances and the lower probability of "foreign substance" rejections which are achieved by the present humanized immunoglobulins of this invention, it is possible and may be felt desirable by the treating physician to administer substantial excesses of these immunoglobulins. Typical prophylactic uses are treatment of a patient undergoing balloon angioplasty to prevent abrupt closure, and of patients who have had one heart attack or stroke to prevent further attacks.

Single or multiple administrations of the compositions can be carried out with dose levels and pattern being selected by the treating physician. In any event, the pharmaceutical formulations should provide a quantity of the immunoglobulin(s) of this invention sufficient to effectively treat the patient.

In particular embodiments, compositions comprising a humanized immunoglobulin of the present invention may be used to diagnose the presence and location of a thrombus in a human patient. For example but not for limitation, the antigenic determinant bound by the mouse antibody C4G1 is present on platelets but is absent or present in reduced amounts on vascular endothelial cells. Thus, a humanized immunoglobulin of the present invention, such as a humanized immunoglobulin that binds to the antigenic determinant identified by the mouse antibody C4G1, may be labeled and used to identify anatomical sites that contain significant concentrations of activated platelets, for example, thrombus sites. For example but not for limitation, one or more labeling moieties may be attached to the humanized immunoglobulin. Exemplary labeling moieties include, but are not limited to, radiopaque dyes, radiocontrast agents, fluorescent molecules, spin-labeled molecules, enzymes, or other labeling moieties of diagnostic value, particularly in radiologic or magnetic resonance imaging techniques.

Humanized immunoglobulins of the present invention can further find a wide variety of utilities in vitro. By way of example, the immunoglobulins can be utilized for detection of GPIIb/IIIa antigens, for isolating platelets, or the like.

For diagnostic purposes, the immunoglobulins may either be labeled or unlabeled. Unlabeled immunoglobulins can be used in combination with other labeled antibodies (second antibodies) that are reactive with the humanized immunoglobulin specific for human immunoglobulin constant regions. Alternatively, the immunoglobulins can be directly labeled. A wide variety of labels may be employed, such as radionuclides, fluors, enzymes, enzyme substrates, enzyme cofactors, enzyme inhibitors, ligands (particularly haptens), etc. Numerous types of immunoassays are available and are well known to those skilled in the art.

Kits can also be supplied for use with the subject immunoglobulins in protection against or detection of a cellular activity or for the presence of a selected antigen. Thus, the subject immunoglobulin composition of the present invention may be provided, usually in a lyophilized form in a container, either alone or in conjunction with additional antibodies specific for the desired cell type. The immunoglobulins, which may be conjugated to a label or toxin, or unconjugated, are included in the kits with buffers, such as Tris, phosphate, carbonate, etc., stabilizers, preservatives, inert proteins, e.g., serum albumin, or the like, and a set of instructions for use. Generally, these materials will be present in less than about 5% based on the amount of active immunoglobulin, and usually present in total amount of at least about 0.001% wt. based again on the immunoglobulin concentration. Frequently, it will be desirable to include an inert extender or excipient to dilute the active ingredients, where the excipient may be present in from about 1 to 99% wt. of the total composition. Where a second antibody capable of binding to the immunoglobulin is employed in an assay, this will usually be present in a separate vial. The second antibody is typically conjugated to a label and formulated in an analogous manner with the immunoglobulin formulations described above.

The following examples are offered by way of illustration.

### EXPERIMENTAL

### Cloning of heavy chain and light chain cDNA

cDNAs for the heavy chain and light chain variable domain genes were cloned using anchored polymerase chain reactions (E.Y. Loh et al., Science, 243, 217 (1989)), using 3' primers that hybridized to the constant regions and contained HindIII sites, and 5' primers that hybridized to the dG tails and contained EcoRI sites (scheme shown in Fig. 1). The PCR amplified fragments were digested with EcoRI and HindIII and cloned into the pUC18 vector for sequencing. For C4G1, two gamma-1 specific and two kappa specific clones were sequenced. The two gamma-1 clones and two kappa clones are respectively identical in sequence. The cDNA variable domain sequences and the deduced amino acid sequences are shown in Fig. 2.

### Construction and expression of chimeric antibody

Two plasmid vectors were prepared for construction and expression of the chimeric antibody genes. The plasmid pVg1-dhfr (Fig. 3A) contains a human cytomegalovirus IE1 promoter and enhancer (M. Boshart et al., Cell, 41, 521 (1985)), the human genomic Cγ1 segment including part of the preceding intron, and a dihydrofolate reductase (dhfr) gene (Simonsen et al., Proc. Natl. Acad. Sci. USA, 80, 2495 (1984), which is incorporated herein by reference) for selection. The plasmid pVk (Fig. 3B) is similar to pVg1-dhfr but contains the human genomic C_{κ} segment and the gpt gene. Derivatives of the C4G1 heavy and light chain variable regions were prepared from the cDNAs by polymerase chain reaction. The 5' primers hybridized to the V regions starting at the ATG codons and contained XbaI sites; the 3' primers hybridized to the last 15 nucleotides of the J regions and contained splice donor signals and XbaI sites (see, Queen et al., Proc. Natl. Acad. Sci. USA, 86, 10029 (1989), which is incorporated herein by reference). The modified V regions were cloned into the XbaI sites of the respective plasmid vectors between the CMV promoter and the partial introns of the constant regions.

For expression of the chimeric antibody, the heavy chain and kappa chain plasmids were transfected into Sp2/0 mouse myeloma cells by electroporation and cells were selected for gpt expression. Clones secreting a maximal amount of complete antibody were detected by ELISA. Purified chimeric C4G1 antibody was shown to bind to HEL 92.1.7 cells, which express the GPIIb/IIIa antigen, by flow cytometry (Fig. 4).

### Computer modeling of humanized antibodies

In order to retain high binding affinity in the humanized antibodies, the general procedures of Queen et al. were followed (see, Queen et al., Proc. Natl. Acad. Sci. USA, 86, 10029 (1989) and WO 90/07861, which are incorporated herein by reference). The more homologous a human antibody is to the original murine antibody, the less likely will combining the murine CDR's with the human framework be to introduce distortions into the CDR's that could reduce affinity. Normally the heavy chain and light chain from the same human antibody are chosen to provide the framework sequences, so as to reduce the possibility of incompatibility in the assembling of the two chains. Based on sequence homology search against the NBRF protein sequence database (performed with the MicroGenie Sequence Analysis Software (Beckman)), the antibody Eu was chosen to provide the framework sequences for humanization of C4G1.

The computer program ENCAD (M. Levitt, J. Mol. Biol., 168, 595 (1983), which is incorporated herein by reference) was used to construct a model of the C4G1 variable region. The model was used to determine the amino acids in the C4G1 framework that were close enough to the CDR's to potentially interact with them (category 4 below). To design the humanized light and heavy chain C4G1 variable regions, at each position the amino acid was chosen to be the same as in the Eu antibody, unless that position fell in one or more of five categories:
(1) The position fell within a CDR,
(2) The Eu amino acid was unusual for human antibodies at that position, whereas the C4G1 amino acid was typical for human antibodies at that position,
(3) The position was immediately adjacent to a CDR,
(4) The model described above suggested that the amino acid may be physically close to the antigen binding region (CDR's).
   In category (2), "unusual" is interpreted to include amino acids that occur in less than about 20% of the human sequences in the same subgroups (as defined by Kabat et al., op. cit.) as the Eu light and heavy chains, and "typical" is interpreted to include amino acids that occur in more than about 25% but generally more than 50% of the human sequences in those subgroups. For positions in these categories, the amino acid from the mouse C4G1 antibody was used. In addition, a position was in the fifth category if
(5) The Eu amino acid was highly unusual for human antibodies at that position, and the C4G1 amino acid was different but also unusual.
Then an amino acid typical for human antibodies at that position may be used.

The amino acids in each category are shown in Table 1. Some amino acids may be in more than one category. The final sequences of the humanized C4G1 light and heavy chain variable domains are shown in Fig. 5A and Fig. 5B, compared with the mouse C4G1 sequences.

**TABLE 1**

| Category | Light Chain | Heavy Chain |
|---|---|---|
| 1 | 24-34, 50-56, 89-97 | 31-35, 50-66, 99-108 |
| 2 | 48, 63 | 93, 98, 109, 110, 113 |
| 3 | ― | 30, 98, 109 |
| 4 | 7, 22, 48, 70, 71, 87 | 27, 28, 30, 48, 70, 72, 96, 109 |
| 5 | ― | 111 |

### Synthesis of humanized antibody

For the construction of genes for the humanized antibodies, nucleotide sequences were selected that encode the protein sequences of the humanized heavy and light chains, including typical immunoglobulin signal peptides, generally utilizing codons found in the mouse sequence. Several degenerate codons were changed to create restriction sites or to remove undesirable ones. The nucleotide sequences also included the same splice donor signals used in the chimeric genes and an XbaI site at each end. Each gene was constructed from four overlapping synthetic oligonucleotides. For each variable domain gene, two pairs of overlapping oligonucleotides on alternating strands were synthesized that encompassed the entire coding sequences as well as the signal peptide and the splice donor signal (Fig. 6). The oligonucleotides were synthesized on an Applied Biosystems 380B DNA synthesizer. Each oligonucleotide was about 110-140 bases long with about a 15 base overlap. Double stranded DNA fragments were synthesized with Klenow polymerase from each pair of oligonucleotides, digested with restriction enzymes, ligated to the pUC18 vector and sequenced. Two fragments with the respectively correct half-sequences were then ligated into the XbaI sites of the pVgl-dhfr or pVk expression vectors in the appropriate orientations to produce the complete heavy and light chain genes. For example, the humanized C4G1 variable domain gene was then contained on an XbaI fragment in pVg1-dhfr. Reactions were carried out under conditions well-known in the art (Maniatis et al., op. cit.). The expected amino acid sequences of the complete light and heavy chains of the humanized C4G1 antibody are shown in Figs. 5C and 5D.

The heavy chain and light chain plasmids were transfected into Sp2/0 mouse myeloma cells by electroporation and cells were selected for gpt expression. Clones were screened by assaying human antibody production in the culture supernatant by ELISA, and antibody was purified from the best-producing clones. Antibody was purified by passing tissue culture supernatant over a column of staphylococcal protein A-Sepharose CL-4B (Pharmacia). The bound antibody was eluted with 0.2 M Glycine-HCl, pH 3.0 and neutralized with 1 M Tris pH 8.0. The buffer was exchanged into PBS by passing over a PD10 column (Pharmacia).

### Synthesis of humanized Fab and F(ab')₂ fragments

Two additional vectors were constructed in order to synthesize the humanized Fab and F(ab')₂ fragments (Figs. 7A and 7B). To distinguish the recombinant F(ab')₂ fragment produced directly using the vector from an F(ab')₂ fragment produced by enzymatic cleavage, the recombinant fragment will be designated F(ab')₂-1. From the XbaI site counterclockwise to the BamHI site, these plasmids are the same as pVg1-dhfr (Fig. 3A). However, instead of the complete human Cγ1 gene, the plasmid pHFab.D contains only the C_{H}1 exon and the first 5 amino acids of the hinge exon, followed by a stop codon and splice donor signal, and the plasmid pHF(ab')2.D contains only C_{H}1, the hinge and the first 2 amino acids of C_{H}2 (counting the codon that bridges the splice as part of the hinge), followed by a stop codon and splice donor signal. In both plasmids, the final exon is followed by a 162-bp SalI-BamHI fragment, which was taken from the region immediately following the C_{H}3 exon of the mouse γ2a constant region gene and which contains a polyadenylation signal. The XbaI fragment containing the humanized C4G1 heavy chain variable domain gene, described above, was then inserted in the appropriate orientation into the XbaI sites of each of the plasmids pHFab.D and pHF(ab')2.D. All plasmids were constructed by standard methods, including oligonucleotide synthesis and polymerase chain reaction, well known to those skilled in the art of genetic engineering. The expected amino acid sequences of the heavy chains in the humanized C4G1 Fab and F(ab')₂-1 fragments are shown in Figs. 7C and 7D; the light chain in these fragments is the same as in the complete antibody (Fig. 5C).

Each heavy chain containing plasmid was respectively transfected into Sp2/0 cells together with the humanized C4G1 light chain containing plasmid by electroporation, and cells were selected for gpt expression. Clones were screened by assaying for human antibody fragments in the culture supernatants by ELISA, and humanized C4G1 antibody Fab or F(ab')₂-1 fragment was purified from the best-producing clones. For each fragment, concentrated culture supernatant was first passed through a DEAE-Sepharose column in 20 mM Tris, pH 8.6, and the flow-through fraction was used for further purification. The Fab fragment was further purified by chromatography on CM-Sepharose and Phenyl-Sepharose, while the F(ab')₂-1 fragment was further purified by chromatography on CM-Sepharose, and Phenyl-Sepharose and on S-200, to separate F(ab')₂-1 from other oligomers of Fab', using methods well known in the art. Other art-known methods of protein preparation and purification may also be applied. For example, the DEAE-Sepharose flow-through fraction of F(ab')₂-1 can be incubated in 10 mM reduced glutathione at pH 9.0 to increase the yield of F(ab')₂-1.

The humanized C4G1 antibody and fragments may also be purified in other, slightly or substantially different ways. In each case, the cells are removed from the culture supernatant by centrifugation at 10,000 g for 10 min, the supernatant is filtered using a 0.45 µm filter, and then concentrated about 20-fold using a membrane with molecular weight cut-off (MWCO) of 30,000 for whole antibody and 10,000 for the fragments. For example, to prepare whole humanized C4G1 antibody, concentrated culture supernatant was then adjusted to pH 7.5 with 1 M Tris, centrifuged at 10,000 g for 10 min and filtered through a 0.45 µm filter. The sample was then loaded on a Protein A-Sepharose FF column (Pharmacia), using 0.15 M NaCl, 0.05 M Tris, pH 7.5, 2 mM EDTA as the equilibrium and wash buffer. The antibody was eluted with 0.1 M acetic acid adjusted to pH 3.5 with Tris base, and the pooled fractions were adjusted to pH 7.5 with Tris. The pool was then dialyzed against PBS using sterile dialysis tubing and filter sterilized using a 0.2 µm filter.

To purify the humanized C4G1 Fab fragment for ex vivo experiments in monkeys, the concentrated culture supernatant was diafiltered against 20 mM Tris, pH 8.6, centrifuged at 10,000 g for 10 min and filtered through a 0.45 µm filter. The sample was applied to a DEAE Sepharose (Pharmacia) column equilibrated with 10 mM Tris, pH 8.6, and the flow-through was collected. The DEAE pool was made 10 mM in MES and adjusted to pH 6.5 with HCl. The sample was then applied to a CM Sepharose (Pharmacia) column equilibrated with 10 mM MES, pH 6.5, and eluted with a 0 to 500 mM NaCl gradient. The Fab fragment was contained in the first peak. The pool of peak fractions was concentrated using a 10,000 MWCO membrane and loaded on a Sephacryl S-200 (Pharmacia) column equilibrated with PBS. The peak fractions were pooled and filter sterilized using a 0.2 µm filter.

The humanized C4G1 F(ab')₂-1 fragment can be prepared and purified as follows. The concentrated culture supernatant is diafiltered against 20 mM Tris, pH 8.6, centrifuged at 10,000 g for 10 min and filtered through a 0.45 µm filter. The sample is applied to a DEAE Sepharose (Pharmacia) column equilibrated with 10 mM Tris, pH 8.6, and the flow-through is collected. The DEAE pool is concentrated about 10-fold using 10,000 MWCO membrane and adjusted to pH 5.0 with acetic acid, and the precipitate is removed by centrifugation. The sample is then adjusted to pH 9.0 with Tris, and 10 mM reduced glutathione is added and incubated for 30 min at 22°C. The sample is then dialyzed for 16 hr at 4°C against 0.1 M Tris, pH 9.0, which results in about 30% F(ab')₂-1 formation. The sample is then concentrated about 10-fold using a 10,000 MWCO membrane and loaded onto a Sephacryl S-200 column. The 90 kDa peak is collected and filter sterilized using a 0.2 µm filter.

Cell lines that produce higher levels of the humanized C4G1 antibody and fragments are obtained by incubating producing cell lines in increasing concentrations of methotrexate (Simonsen et al., op. cit.). Alternatively, Fab and F(ab')₂ fragments can be prepared by enzymatic cleavage of intact humanized C4G1 antibody using methods well known in the art (Antibodies. A Laboratory Manual, Harlow and Lane, Eds., Cold Spring Harbor Laboratory, 1988, which is incorporated herein by reference), instead of by recombinant methods as above.

For example, another form of the humanized C4G1 F(ab')₂ fragment, designated F(ab')₂-2, was prepared by pepsin digestion as follows. The culture supernatant of the humanized C4G1 IgG producing cells was adjusted to pH 8.5 with 1 M Tris, and applied to a protein A affinity membrane device (Nygene) equilibrated with 50 mM Tris pH 8.5, 150 mM NaCl, 2 mM EDTA. The whole antibody was eluted with 0.1 M Glycine-HCl pH 2.5, and the pooled fractions were adjusted to pH 3.5 with 1 M Tris. Thus prepared IgG was digested by pepsin from Porcine Stomach Mucosa (Sigma). The pepsin was dissolved in 0.1 M Glycine-HCl pH 3.5 and added to the IgG solution (the enzyme: IgG ratio (w/w) should be 1:100). The reaction mixture was incubated with inverting for 3 hrs. at 37°C, and the reaction was stopped by addition of 1 M Tris to adjust to pH 8.0. The pepsin digested fraction was then adjusted to 0.75 M (NH₄)₂SO₄, and applied to a Phenyl-5PW (Tosoh) column equilibrated with 50 mM CH₃COONa pH 6.0, 0.75 M (NH₄)₂SO₄. The sample was eluted with a linear gradient from 0.75 M to 0 M (NH₄)₂SO₄. The F(ab')₂-2 was contained in the main peak.

### Properties of humanized antibodies

The humanized C4G1 antibody was characterized in comparison to the murine and chimeric antibodies. The humanized antibody bound to HEL 92.1.7 cells in a fluorocytometric analysis in a manner similar to the chimeric antibody (Fig. 4), showing that it recognizes the GPIIb/IIIa antigen.

Purified humanized C4G1 antibody, Fab fragment, F(ab')₂-1 fragment and F(ab')₂-2 fragment were reduced and run on an SDS PAGE gel. The whole antibody showed 2 bands of approximate molecular weights 25 kDa and 50 kDa, and the Fab and F(ab')₂-1 fragments showed a doublet of bands of approximately 25 kDa. The humanized C4G1 F(ab')₂-2 fragment showed the same mobility as the F(ab')₂-1 fragment on SDS-PAGE. These results are consistent with the molecular weights of the light chain and heavy chain or heavy chain fragment calculated from their amino acid compositions.

The affinities of the humanized C4G1 antibody, Fab fragment and F(ab')₂-1 fragment were determined by competitive binding with the respective mouse C4G1 antibody and fragments. Purified human platelets were used as source of the GPIIb/IIIa antigen. The platelets were purified from buffy coats from normal human donors by centrifugation on ficoll-hypaque. Increasing amounts of humanized or mouse competitor antibody or fragment was added to 4.5 ng of radioiodinated tracer C4G1 antibody (3.5 µCi/µg) and incubated with 3×10⁷ platelets in 0.2 ml of modified Tyrode's buffer for 1 hr. at room temperature. Cells were washed with 2 ml of ice-cold buffer and pelleted. The radioactivities were measured, and the concentrations of bound and free tracer antibody were calculated (Fig. 8). The binding affinities of the various antibodies and fragments were calculated as in Queen et al., Proc. Nat. Acad. Sci. USA, 86, 10029 (1989). The results clearly show that there is no significant difference in binding affinities between respectively the humanized and mouse C4G1 antibodies (Fig. 8A), the humanized and mouse C4G1 Fab fragments (Fig. 8B), and the humanized and mouse C4G1 F(ab')₂ fragments (Fig. 8C). The same result was obtained when competitive binding was performed with HEL 92.1.7 cells as source of GPIIb/IIIa antigen. Moreover, all the actual binding affinities to these cells were about 10⁸ M⁻¹ or higher.

The ability of the mouse and humanized C4G1 antibodies and fragments to inhibit platelet aggregation was determined. Each antibody or fragment was added to platelet rich plasma (PRP), prepared by centrifuging freshly drawn citrated blood at 200 × g for 10 min., at a final concentration of 5 µg/ml and incubated for 5 min. with stirring at 37°C. ADP was added to a final concentration of 4.6 µM to initiate aggregation. Platelet aggregation was monitored by light transmission, using a Sienco dual sample aggregation meter, model DP-247F or HEMA-TRACER. There were no significant differences in the ability of the humanized and mouse C4G1 antibody to inhibit aggregation (Fig. 9) or of the respective humanized and mouse Fab and F(ab')₂-1 fragments to inhibit aggregation. And there were no significant differences in the ability of the humanized C4G1 F(ab')₂-1 and F(ab')₂-2 fragments to inhibit aggregation.

Binding of the murine and the humanized C4G1 antibodies to human umberical vein endothelial cells (HUVEC) was examined in comparison with binding of a murine monoclonal antibody B6A3, which is specific to GPIIIa. Binding of the antibodies to HEL 92.1.7 cells was also examined. The antibodies were radiolabeled (3.5 µCi/µg) as described above. Suspensions of 500 µl HUVEC or HEL 92.1.7 (2×10⁴/ml, 2×10⁵/ml, 2×10⁶/ml) were incubated for 90 min with 100 fmole of each monoclonal antibody at room temperature or at 4°C in 1.5 ml Eppendorf tubes. After incubation the tube contents were layered onto silicone oil in Eppendorf centrifuge tubes and spun at 10,000 × g for 2 min at room temperature. The pellet and supernatant were counted separately in a gamma counter. Nonspecific binding was measured at a 100-fold excess of unlabeled antibody and subtracted from the total bound to give specific binding. Both the murine and the humanized C4G1 monoclonal antibodies showed specific binding to HEL 92.1.7 cells as well as the murine B6A3 at the cell density 2×10⁵/ml and 2×10⁶/ml. In contrast, neither the murine C4G1 nor the humanized C4G1 did show specific binding to HUVEC at any cell densities whereas the murine B6A3 bound specifically to HUVEC at 2×10⁶/ml.

### Ex vivo inhibitory activity of ADP-induced platelet aggregation

The ability of the humanized C4G1 Fab and F(ab')₂ fragments to inhibit ex vivo platelet aggregation from monkeys was determined. Although platelets from all healthy human volunteers responded to humanized C4G1, the platelets from some rhesus monkeys did not respond to humanized C4G1. Therefore, only rhesus monkeys with platelets responding to humanized C4G1 were chosen for this experiment.

Each fragment was suspended in 5 ml saline solution and administered I.V. to rhesus monkeys (male, 2.8-5.0 kg weight). In the placebo control, 5 ml saline solution was administered. Blood was collected 1 hr after administration under anesthesia with ketamine hydrochloride, and immediately citrated and centrifuged at 200 g for 10 min. The supernatant was used as PRP (platelet-rich plasma). Platelet-poor plasma (PPP) was prepared from the remaining blood after PRP was removed by centrifuging the blood at 2,000 g. PRP was diluted with PPP to achieve a platelet count of 3×10⁸/ml.

ADP was added to a final concentration of 20 µM to initiate aggregation. Platelet aggregation was monitored by light transmission.

Platelet aggregation was completely inhibited in the platelets obtained from monkeys treated with the humanized C4G1 Fab and F(ab')₂-1 fragments. The F(ab')₂-1 fragment completely inhibited platelet aggregation at a dose of 0.5 mg/kg body weight. Treatment with the fragments did not cause significant thrombocytopenia in the monkeys.

From the foregoing, it will be appreciated that the humanized immunoglobulins of the present invention offer numerous advantages over other GPIIb/IIIa specific antibodies. In comparison to mouse monoclonal antibodies, the present humanized immunoglobulins contain substantially less foreign amino acid sequences. This reduced likelihood of antigenicity after injection into a human patient represents a significant therapeutic improvement.

All publications and patent applications are herein incorporated by reference to the same extent as if each individual publication or patent application was specifically and individually indicated to be incorporated by reference. Although the present invention has been described in some detail by way of illustration and example for purposes of clarity and understanding, it will be apparent that certain changes and modifications may be practiced within the scope of the appended claims.

## Claims

1. A humanized immunoglobulin having the properties:
[1] an epitope for said humanized immunoglobulin exists in a glycoprotein GPIIb/IIIa on human platelets,
[2] an epitope for said humanized immunoglobulin does not exist in human vascular endothelial cells,
[3] said humanized immunoglobulin has a binding affinity of 10⁷ M⁻¹ or stronger, and
[4] said humanized immunoglobulin is capable of blocking the aggregation of human platelets in response to agonists,
wherein said humanized immunoglobulin comprises a sequence which is the mature light variable region of the upper sequence in Figure 5A and a sequence which is the mature heavy variable region of the upper sequence in Figure 5B.

2. A humanized immunoglobulin according to claim 1 wherein said humanized immunoglobulin comprises a sequence which is the mature light region as shown in Figure 5C and a sequence which is the mature heavy region as shown in Figure 5D.

3. A humanized immunoglobulin which is an Fab or an F(ab')₂ obtainable by the enzymatic digestion of humanized immunoglobulin according to claim 1 or 2.

4. A therapeutic agent for treating thromboembolic diseases, said agent comprising humanized immunoglobulin according to claim 1 or 2 or 3.

5. A polynucleotide which comprises a sequence coding for said mature variable light region of humanized immunoglobulin according to claim 1, or a sequence coding for said mature heavy variable region of humanized immunoglobulin according to claim 1.

6. A polynucleotide which comprises a sequence coding for said mature light region of humanized immunoglobulin according to claim 2, or a sequence coding for said mature heavy region of humanized immunoglobulin according to claim 2.

7. An expression vector comprising a polynucleotide according to claim 5 or 6.

8. A cell transfected with an expression vector according to claim 7.

9. A cell transfected with two expression vectors according to claim 7 wherein the first vector comprises a sequence coding for a mature light chain region and the second vector comprises a sequence coding for a mature heavy chain region.

10. A process for producing a humanized immunoglobulin according to claim 1 or 2 which comprises :
culturing a cell according to claim 8 or 9, and
collecting humanized immunoglobulin from the culture.

11. A process for producing a humanized immunoglobulin according to claim 1 or 2 or 3 which comprises :
culturing a cell according to claim 8 or 9,
collecting humanized immunoglobulin from the culture, and
digesting the resulting humanized immunoglobulin with an enzyme,
wherein said humanized immunoglobulin is an Fab or an F(ab')₂.

## Patentansprüche

1. Humanisiertes Immunglobulin mit den folgenden Eigenschaften:
[1] ein Epitop für das genannte humanisierte Immunglobulin existiert in einem Glykoprotein GPIIb/IIIa auf menschlichen Blutplättchen,
[2] ein Epitop für das genannte humanisierte Immunglobulin existiert nicht in menschlichen Gefäßendothelzellen,
[3] das genannte humanisierte Immunglobulin hat eine Bindungsaffinität von 10⁷ M⁻¹ oder stärker, und
[4] das genannte humanisierte Immunglobulin kann die Aggregation menschlicher Blutplättchen als Reaktion auf Agonisten blockieren,
wobei das genannte humanisierte Immunglobulin eine Sequenz, die die reife leichte variable Region der oberen Sequenz in Fig. 5A ist, und eine Sequenz umfasst, die die reife schwere variable Region der oberen Sequenz in Fig. 5B ist.

2. Humanisiertes Immunglobulin nach Anspruch 1, wobei das genannte humanisierte Immunglobulin eine Sequenz, die die reife leichte Region ist (siehe Fig. 5C), und eine Sequenz umfasst, die die reife schwere Region ist (siehe Fig. 5D).

3. Humanisiertes Immunglobulin, das ein Fab oder ein F(ab')₂ ist, erhältlich durch die enzymatische Verdauung von humanisiertem Immunglobulin nach Anspruch 1 oder 2.

4. Therapeutisches Mittel zur Behandlung thromboembolischer Krankheiten, wobei das genannte Mittel ein humanisiertes Immunglobulin nach Anspruch 1, 2 oder 3 umfasst.

5. Polynucleotid, das eine Sequenzkodierung für die genannte reife variable leichte Region von humanisiertem Immunglobulin nach Anspruch 1 oder eine Sequenzkodierung für die genannte reife schwere variable Region von humanisiertem Immunglobulin nach Anspruch 1 umfasst.

6. Polynucleotid, das eine Sequenzkodierung für die genannte reife leichte Region von humanisiertem Immunglobulin nach Anspruch 2 oder eine Sequenzkodierung für die genannte reife schwere Region von humanisiertem Immunglobulin nach Anspruch 2 umfasst.

7. Expressionsvektor, umfassend ein Polynucleotid nach Anspruch 5 oder 6.

8. Zelle, transfiziert mit einem Expressionsvektor nach Anspruch 7.

9. Zelle, transfiziert mit zwei Expressionsvektoren nach Anspruch 7, wobei der erste Vektor eine Sequenzkodierung für eine reife leichte Kettenregion und der zweite Vektor eine Sequenzkodierung für eine reife schwere Kettenregion umfasst.

10. Verfahren zur Herstellung eines humanisierten Immunglobulins nach Anspruch 1 oder 2, umfassend die folgenden Schritte:
Kultivieren einer Zelle nach Anspruch 8 oder 9, und
Sammeln von humanisiertem Immunglobulin von der Kultur.

11. Verfahren zur Herstellung eines humanisierten Immunglobulins nach Anspruch 1, 2 oder 3, umfassend die folgenden Schritte:
Kultivieren einer Zelle nach Anspruch 8 oder 9,
Sammeln von humanisiertem Immunglobulin von der Kultur, und
Verdauen des resultierenden humanisierten Immunglobulins mit einem Enzym,
wobei das genannte humanisierte Immunglobulin ein Fab oder ein F(ab')₂ ist.

## Revendications

1. Immunoglobuline humanisée ayant les propriétés :
[1] un épitope pour ladite immunoglobuline humanisée existe dans une glycoprotéine GPIIb/IIIa sur les plaquettes humaines,
[2] un épitope pour ladite immunoglobuline humanisée n'existe pas dans les cellules endothéliales vasculaires humaines,
[3] ladite immunoglobuline humanisée a une affinité de liaison de 10⁷ M⁻¹ ou plus forte, et
[4] ladite immunoglobuline humanisée est capable de bloquer l'agrégation des plaquettes humaines en réponse aux agonistes,
où ladite immunoglobuline humanisée comprend une séquence qui est la région variable légère mature de la séquence supérieure sur la Figure 5A et une séquence qui est la région variable lourde mature de la séquence supérieure sur la Figure 5B.

2. Immunoglobuline humanisée selon la revendication 1 dans laquelle ladite immunoglobuline humanisée comprend une séquence qui est la région légère mature telle qu'illustrée sur la Figure 5C et une séquence qui est la région lourde mature telle qu'illustrée sur la Figure 5D.

3. Immunoglobuline humanisée qui est un Fab ou un F(ab')₂ qui peut être obtenu par la digestion enzymatique de l'immunoglobuline humanisée selon la revendication 1 ou 2.

4. Agent thérapeutique pour traiter les maladies thrombo-emboliques, ledit agent comprenant une immunoglobuline humanisée selon la revendication 1 ou 2 ou 3.

5. Polynucléotide qui comprend une séquence codant ladite région variable légère mature de l'immunoglobuline humanisée selon la revendication 1, ou une séquence codant ladite région variable lourde mature de l'immunoglobuline humanisée selon la revendication 1.

6. Polynucléotide qui comprend une séquence codant ladite région légère mature de l'immunoglobuline humanisée selon la revendication 2, ou une séquence codant ladite région lourde mature de l'immunoglobuline humanisée selon la revendication 2.

7. Vecteur d'expression comprenant un polynucléotide selon la revendication 5 ou 6.

8. Cellule transfectée avec un vecteur d'expression selon la revendication 7.

9. Cellule transfectée avec deux vecteurs d'expression selon la revendication 7 dans laquelle le premier vecteur comprend une séquence codant une région mature à chaîne légère et le deuxième vecteur comprend une séquence codant une région mature à chaîne lourde.

10. Procédé pour produire une immunoglobuline humanisée selon la revendication 1 ou 2 qui comprend :
cultiver une cellule selon la revendication 8 ou 9, et
collecter l'immunoglobuline humanisée de la culture.

11. Procédé pour produire une immunoglobuline humanisée selon la revendication 1 ou 2 ou 3 qui comprend :
cultiver une cellule selon la revendication 8 ou 9,
collecter l'immunoglobuline humanisée de la culture, et
digérer l'immunoglobuline humanisée obtenue avec une enzyme,
dans lequel ladite immunoglobuline humanisée est un Fab ou un F(ab')₂.
